# EUROPEAN PATENT APPLICATION

(11) **EP 1 595 683 A2**
(43) Date of publication of application: **16.11.2005**
(21) Application number: 05009563.7
(22) Date of filing: 02.05.2005
(51) Int. Cl.: B29C 49/20, A61F 5/451

(54) **Blow moulding a thermoplastic on an insert for producing a part of a ostomy device**

(30) Priority: 10.05.2004 US 569543 P
(71) Applicant: Bristol-Myers Squibb Company, New York, N.Y. 10154 (US)
(72) Inventor: Blum, John L., Toms River New Jersey 08757 (US)
(74) Representative: Holmes, Miles Keeton

(57) **Abstract**

A method and apparatus for forming an ostomy device component, for attaching a preformed first portion (14) of the component to a second portion (16). The second portion (16) is formed by being blow molded into intimate bonding contact with the preformed first portion (14). The preformed first portion may be injection molded. An ostomy device component formed by this technique may comprise first (14) and second (16) portions, at least one of which is a blow molded part integrally molded to the other part.

## Description

### Field of the Invention

The present invention may relate to the field of plastics molding, for example, for an ostomy device. Particular aspects of the invention may relate to a molding method, molding apparatus, and to a molded product usable in an ostomy device. The invention may be especially applicable to blow molding a component of an ostomy device.

### Background to the Invention

In the technically specialized field of ostomy devices, fusion is a commonly used technique for bonding together two plastics components. Examples of fusion techniques include solvent bonding, heat welding and ultrasonic welding. These techniques generally involve at least partly melting and fusing the two components together after the components have been formed as separate parts. However, such processes necessitate additional manufacturing equipment and processing steps, and add to the cost and complexity of manufacture. There is also a risk of failure of, or leakage from, the weld, resulting from occasional welding errors or anomalies. The manufacturing complexity and risk of failure may be exacerbated when one or both of the components is a blow molded component. A blow molded component typically has a thin wall that may be at least partly deformable, and this can make welding complicated shapes difficult. Should a failure or leak occur during use by an ostomate, not only is this highly embarrassing and unhygienic for the ostomate, it may also lead to customer rejection of the product in this highly personal field.

### Summary of the Invention

Broadly speaking, in one aspect of the invention, a method of forming an ostomy device component may include blow molding a (second) portion of the component into intimate contact with a preformed (first) portion.

The intimate contact achieved during the blow molding operation can enable a strong and permanent bond to be achieved between the two portions, without requiring any additional welding or other fusion step after the molding operation. The blow molding operation may thus achieve two simultaneous effects of (i) imparting a desired shape to the blow molded (second) portion, and (ii) integrally bonding the two portions together.

The invention can therefore avoid the additional welding steps of the conventional manufacturing techniques, leading to improved quality components, and more efficient manufacture.

As used herein, the term "blow molding" may mean any molding process that uses a pressure differential between an internal pressure and an external pressure, to cause a plastics body (e.g. parison) to expand and to adopt a shape define by a surrounding mold.

The material from which the second portion is blow molded may be a body of material that is independent of the first portion. The material from which the second portion is blow molded may, prior to the blow molding operation, be unattached to the first portion.

The first portion may be any preformed part. For example, the first portion may be preformed by injection molding, blow molding, vacuum forming, machining or casting. The first portion could alternatively comprise any of a fabric, textile or non-woven polymer material. The first portion may be a molded part or a sheet material, for example, plastics film. The first portion may itself be a subassembly of multiple parts.

The first portion may be generally rigid (or at least more rigid than the second portion). Alternatively, the first portion may be substantially flexible (or at least more flexible than the second portion).

The first and second portions may be formed of material having substantially the same or similar melting temperatures. Such a technique has been found to provide good bonding between the first and second portions.

The first portion may comprise the same or a similar plastics material to the second portion. Alternatively, the materials used for the first and second portions may be different. The first and second portions may achieve a plastics bond, or a mechanical bond, or a combination of both.

Broadly speaking, a second aspect of the invention may relate to a mold apparatus that may be used in the above method. The, or a, mold apparatus may include a first mold region for accommodating and/or preforming a first preformed portion, and a second mold region for defining a blow mold shape of a second portion.

Broadly speaking, a third aspect of the invention may provide an ostomy device component produced by the method of the first aspect and/or the mold apparatus of the second aspect.

Broadly speaking, a fourth aspect of the invention may provide an ostomy device component that includes integrally molded first and second portions, at least one portion (e.g. the second portion) being a blow molded part.

The first and second portions of the component may include any of the aforementioned features.

The first portion may, in one form, comprise a ring-shaped member and/or a cover member. The first portion may be configured as a coupling member for coupling to a counterpart coupling member. Additionally or alternatively, the second portion may, in one form, comprise a generally tubular portion. The tubular portion may be collapsible and/or extendable in a direction generally parallel to the axis of the tubular portion. The tubular portion may have an open end and/or a closed end. The tubular portion may have a concertina and/or bellows profile. The tubular portion may be flexible and/or bendable. The tubular portion may define an ostomy collection chamber.

The above aspects of the invention may be used independently or in combination, as desired.

Other aspects, features and advantages of the invention may be described in the appended claims and/or in the following description and drawings. Protection is claimed for any novel feature and/or combination of features described herein and/or illustrated in the drawings whether or nor emphasis has been placed thereon.

### Brief Description of the Drawings

Non-limiting preferred embodiments of the invention are now described, by way of example only, with reference to the accompanying drawings, in which:
Fig. 1 is a perspective schematic view of a component of an ostomy device;
Fig. 2 is a schematic cross-section through the component of Fig. 1;
Figs. 3a-e are schematic diagrams illustrating molding of the component of Fig. 1;
Fig. 4 is a schematic diagram illustrating a second modified embodiment of an ostomy device component;
Fig. 5 is a schematic section illustrating a mold for forming the component of Fig. 4;
Fig. 6 is a schematic diagram illustrating a third modified embodiment of an ostomy device component;
Fig. 7 is a schematic section illustrating a mold for forming the component of Fig. 6; and
Fig. 8 is a schematic section illustrating a part of a fourth modified embodiment of an ostomy device component.

### Detailed Description of Preferred Embodiments

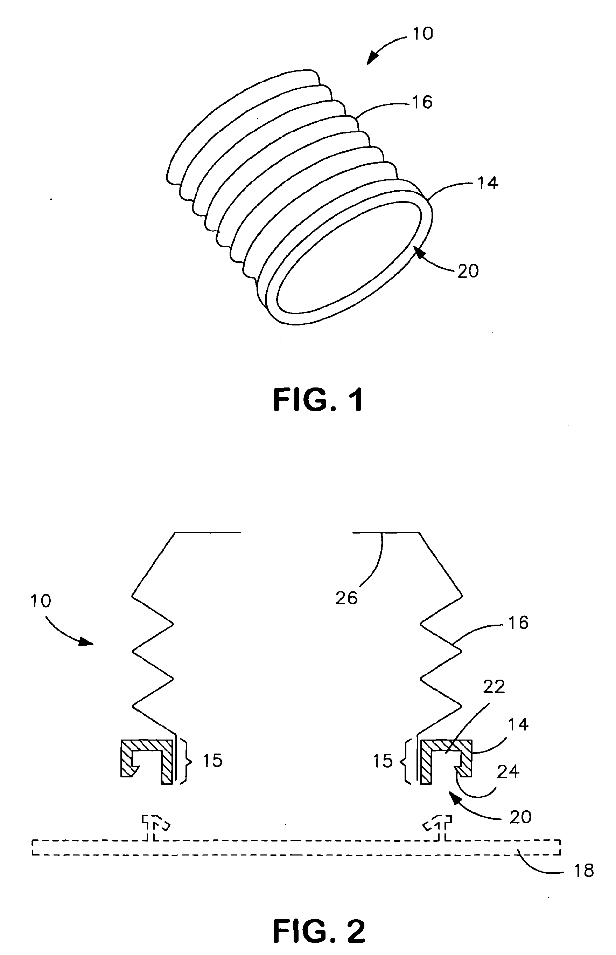

Referring to Figs. 1 and 2, a component 10 for an ostomy device may be shown. The component 10 may define at least partly a chamber for collecting body waste. The component 10 may include a first portion 14 from which extends or depends a second portion 16. The first portion 14 may be generally ring shaped. The first portion 14 may be an injection molded part. The first portion 14 may be partly flexible and/or resiliently deformable, but may generally have a well-defined natural shape. The second portion 16 may be generally tubular. The second portion 16 may be a blow molded part. The second portion 16 may be more flexible than the first portion 14, and may define a concertina bellows capable of being collapsed or expanded in an axial direction. Although the component 10 may be illustrated in Fig. 1 as having a generally circular cross-section shape, any closed-loop shape may be used. One of the first and second portions 14 and 16 may be configured to encircle the other in the region 15 of mutual contact.

The first portion 14 may be configured to support the shape of the tubular second portion 16. The first portion 14 may be configured to provide structural support for the second portion 16. The first portion 14 may additionally or alternatively be configured as a coupling member for coupling to a counterpart annular coupling member (shown in phantom at 18 in Fig. 2). The first portion 14 may be intended to be releasably coupled to the counterpart coupling member 18, to enable the component 10 to be releasably secured to the counterpart member 18. The first portion 14 may, for example, include a coupling configuration 20 for releasable locking engagement with the counterpart member 18. The coupling configuration 20 may include a channel 22 and one or more locking projections or latches 24 that may be undercut relative to the adjacent wall material of the coupling configuration 20.

The first and second portions 14 and 16 may be of material (e.g. plastics) having the substantially the same, or similar, melting temperatures. The first and second portions 14 and 16 may be of the same, or similar, plastics material. For example, the plastics material may be an ethylene vinyl acetate, although a wide variety of similar and dissimilar plastics may be used, as desired.

It may be preferred to form the first portion 14 by injection molding. For example, the first portion 14 may have a relatively thick wall thickness (e.g. about 1 mm, or more) that is better suited to formation by injection molding than blow molding. The coupling configuration 20 of the first portion 14 may also have a level of intricacy that may be suited to injection molding, but may be difficult to achieve by blow molding. In contrast, it may be preferred to form the second portion 16 by blow molding. For example, the second portion 16 may have a relatively thin wall thickness (e.g. about 1 mm or less, typically between 0.1 and 0.3 mm) that may be better suited to formation by blow molding than injection molding. Also, blow molding may enable the second portion 16 to be formed consuming far less plastics material than injection molding, thus leading to significant material economy.

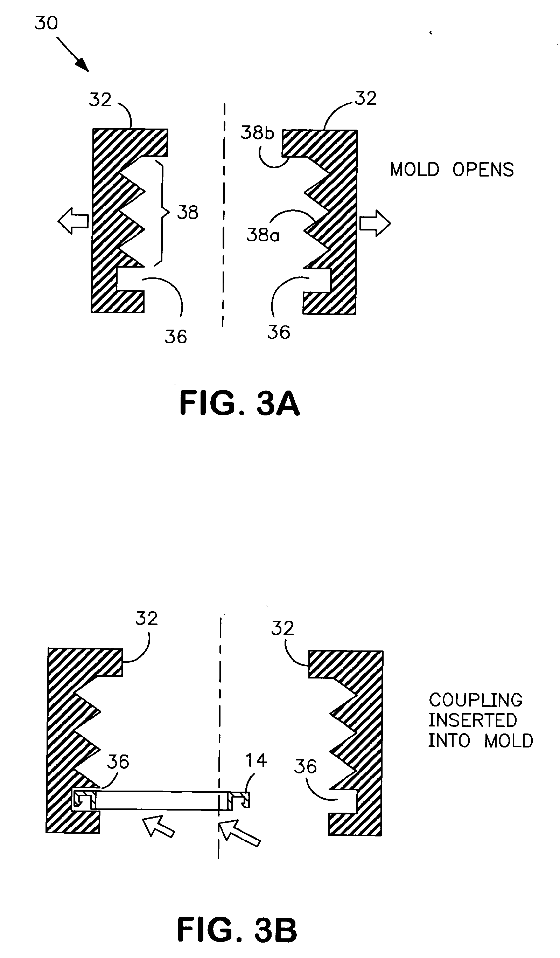

Figs. 3a-e may illustrate a mold apparatus 30 and mold method for forming the component of Figs. 1 and 2. In the present embodiment, the first portion 14 may formed before the second portion 16. The first portion 14 may be preformed in the same mold apparatus as the second portion 16, or the first portion 14 may be preformed in a different mold apparatus (not shown) and transferred to the mold apparatus 30. In the present embodiment, the first portion 14 may have a relatively complicated shape that might best be molded in a separate mold apparatus (or, at least, a separate mold cavity).

The mold apparatus 30 may generally comprise mold segments or shells 32 (e.g. mold halves) that together define a mold cavity 34. The mold shells 32 may include at least one first mold region 36 shaped to accommodate the preformed first portion 14, and at least one second mold region 38 shaped to define the final shape of the second portion 16. In the present embodiment, the first mold region 36 may comprise an annular recess or groove for receiving the preformed ring-shaped first portion 14. The second mold region 38 may comprise a bellows shaped surface 38a for defining the bellows profile of the second portion 16, and a lip surface 38b for defining a radially directed lip 26 of the second portion 16 at the opposite end of the second portion 16 to the first portion 14. The second mold region 38 may be configured to mold the second portion 16 either in a fully extended condition, or in a partly extended condition, or in a fully or substantially collapsed condition.

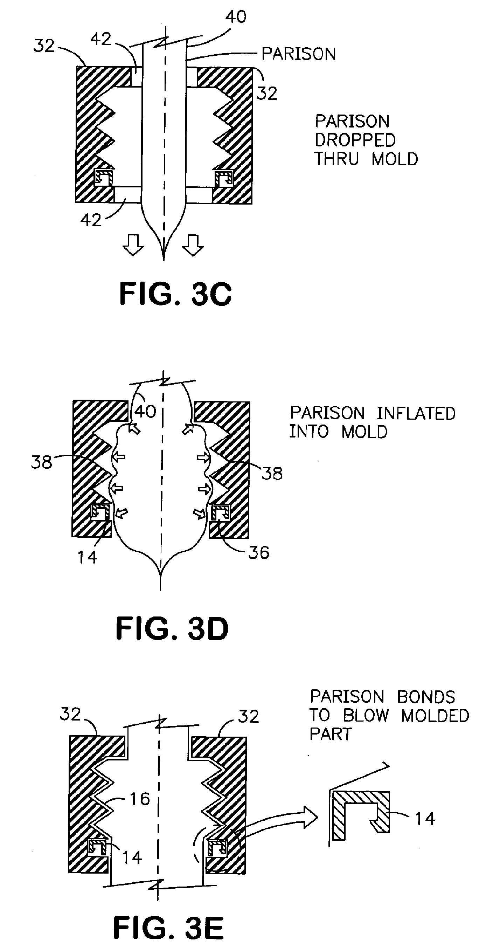

Referring to Fig. 3a, the mold shells 32 may firstly be opened (e.g. moved apart) to allow access to the interior of the cavity. Referring to Fig. 3b, the preformed first portion 14 may be inserted or placed at the first mold region 36, and the mold shells 32 closed (e.g., moved towards each other). Referring to Fig. 3c, an extruded parison 40 may be introduced into the mold apparatus 30 to extend through one or more apertures 42. Referring to Figs. 3d and 3e, the parison 40 may be heated and expanded by blowing gas (e.g. air) into the parison 40 to cause the parison to inflate and adopt the shape defined by the second mold region 38. At the same time, the parison 40 may be forced into intimate contact with the preformed first portion 14 retained in the first mold region 36, to form a secure and permanent mold bond between the first and second portions 14 and 16.

The above technique of blow molding a portion 16 of the component into intimate contact with a preformed portion 14 has been found to provide excellent bonding characteristics between the two portions 14 and 16. The blow molding operation forces the material of the two portions into firm contact, and enables a strong and uniform bond to be achieved with an excellent reliability and repeatability, leading to consistently high manufacturing quality. The quality and reliability may exceed that achievable economically by the prior art technique of welding the two portions together after both portions have been formed as separate parts. Avoiding such a welding step may also improve the manufacturing efficiency because it may no longer be necessary to provide dedicated welding equipment and welding process steps for bonding two discrete portions together.

The blow molding technique may comprise one or more of: extrusion blow molding, injection blow molding, vacuum forming, pressure forming, and/or plug assist thermoforming.

In the illustrated embodiment, the adjoined faces of the first and second portions 14 and 16 may be generally planar. Alternatively, the adjoined faces may be keyed or embossed in order to increase the engagement between the first and second portions 14 and 16, which may increase the strength of the bond.

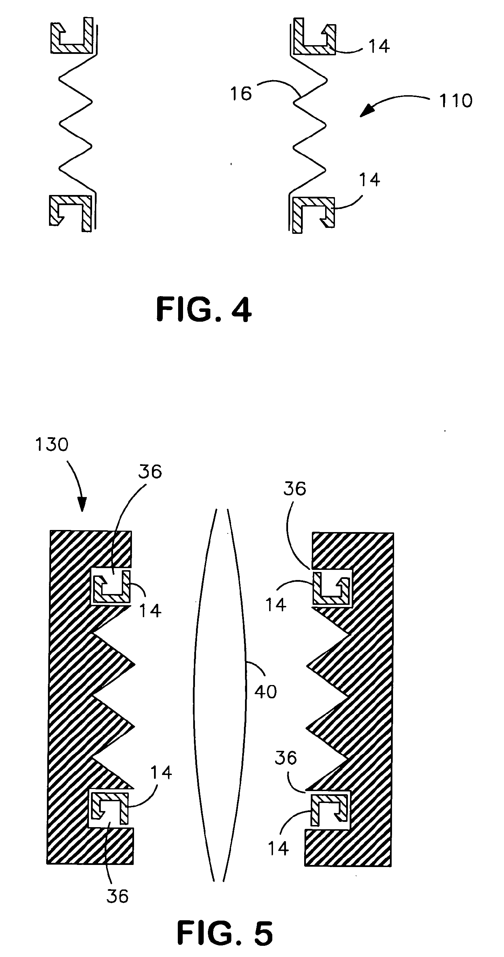

Figs. 4-8, may illustrate modified embodiments of the invention for the purposes of illustration. The same reference numerals may be used where appropriate to identify features already described.

Fig. 4 may illustrate a second form of component 110. The component 110 may be similar to the component 10 described above, except that first portions 14 may be provided at both ends of the second portion 16. Each first portion 14 may be configured as a coupling member for coupling to respective counterpart members (not illustrated in Fig. 4).

Fig. 5 may illustrate a second form of mold apparatus 130 for forming the second component 110. The mold apparatus 130 may include two first mold regions 36 for accommodating the two preformed first portions 14 at opposite ends of the mold cavity 34. The mold apparatus 130 may function in the same manner as the apparatus 30 described above.

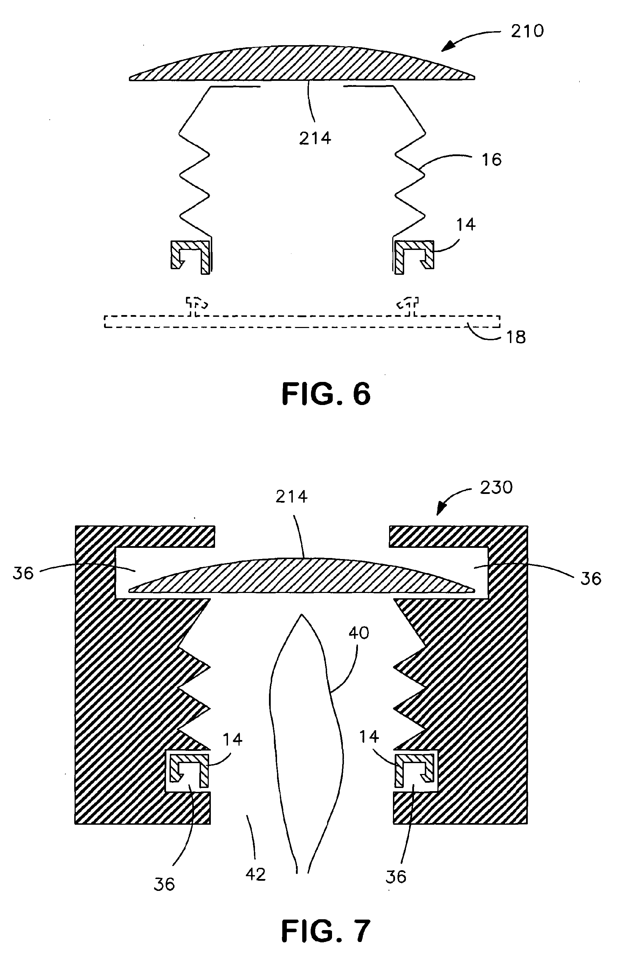

Fig. 6 may illustrate a third form of component 210. The component may be similar to the component 10 described above except that one end of the second portion 16 may be closed by a cover portion 214. The cover portion 214 may be injection molded, similarly to the first portion 14. The cover portion 214 may extend laterally outside the second portion. The cover portion 214 may be configured to releasably engage the counterpart coupling member 18 when the second portion 16 is collapsed down.

Fig. 7 may illustrate a third form of mold apparatus 230 for forming the third component 210. The mold apparatus 230 may include two first mold regions 36 in a similar manner to the apparatus 130 described above. The parison 40 may extend through a single aperture 42, and be configured to be blown into intimate contact with the cover portion 214 as well as the first portion 14, to form a tubular chamber closed at one end.

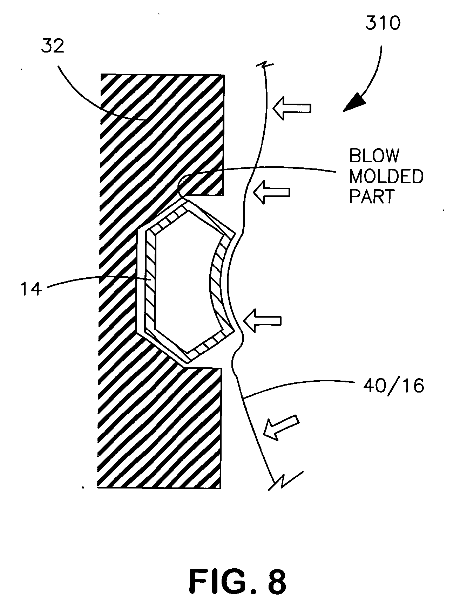

Fig. 8 may illustrate molding of a fourth form of component 310. The component 310 may be similar to the component 10 described above, except that the first portion 14 may be preformed by a technique other than injection molding. Such other techniques may comprise one or more of: blow molded, vacuum formed, machined and/or cast. A further possibility is that the first portion 14 may be of or comprise one or more of: fabric, textile or non-woven polymer material. A yet further possibility is that the first portion 14 may be a sheet material, for example, plastics film.

Although the foregoing embodiments have been described in the form of a component including a ring-shaped first portion 14 and a tubular second portion 16, this is merely for the purposes of illustration. The invention may be used to form a wide variety of different component shapes and configurations in the ostomy field.

The techniques of the present invention are especially advantageous and beneficial in the field of ostomy devices. The manufacture of such devices presents unique challenges in terms of forming low-cost, lightweight devices that are straightforward to use and comfortable to wear. At the same time, security and hygiene are of utmost importance to the wearer. Ostomy devices should have a high integrity against leakage, and be robust enough to withstand body movements and accidental knocks without accidentally releasing body waste. The present invention permits a multi-part component including at least a blow molded part to be integrally formed by a process which is efficient from the point of view of manufacture, yet also achieves the degree of strength and reliability demanded for the specialized field of ostomy devices.

The foregoing description is merely illustrative of preferred forms of the invention. Many modifications, improvements and equivalents may be used within the scope and/or spirit of the invention.

## Claims

1. A method of forming an ostomy device component, comprising:
providing a preformed first portion of the component; and
blow molding a second portion of the component so as to (i) shape the second portion and (ii) force the second portion into intimate contact with the first portion, to bond the first and second portions together.

2. The method according to claim 1, wherein a body of material from which the second portion is blow molded is, prior to the blow molding process, independent of the first portion.

3. The method according to claim 1 or 2, wherein the step of providing the preformed first portion comprises the sub-step of introducing the preformed first portion into first mold region of a mold apparatus for performing the blow molding process.

4. The method according to claim 1 or 2, wherein the step of providing the preformed first portion comprises molding the first portion.

5. The method according to claim 4, wherein the step of molding the first portion comprises injection molding the first portion.

6. The method according to any preceding claim, wherein the first and second portions comprise plastics material of substantially similar melting temperatures.

7. The method according to any preceding claim, wherein the first portion is a subassembly of multiple parts.

8. Molding apparatus for forming an ostomy device component, the molding apparatus comprising a plurality of mold parts defining:
a first molding region for accommodating a preformed first portion of the component; and
a second molding region defining a blow mold shape for forming a second portion of the component in intimate contact with the first portion.

9. The molding apparatus according to claim 8, wherein the mold parts further include an aperture for receiving a blow mold parison independent of the first portion.

10. The molding apparatus according to claim 8 or 9, wherein the first molding region comprises a recessed portion adjacent to the second molding region.

11. An ostomy device component comprising a first portion and a second portion integrally molded to the first portion, wherein the component is formed by a method comprising:
providing the first portion as a preformed part; and
blow molding the second portion of the component so as to (i) shape the second portion and (ii) force the second portion into intimate contact with the first portion, to bond the first and second portions together.

12. An ostomy device component comprising a first and second portions, the second portion being a blow molded part integrally molded to the first portion.

13. The ostomy device component according to claim 11 or 12, wherein the first and second portions are of substantially similar plastics.

14. The ostomy device component according to claim 11, 12 or 13, wherein the first and second portions are of plastics having substantially similar melting temperatures.

15. The ostomy device component according to any of claims 11 to 14, wherein either the first or second portion substantially encircles the other, in the region of mutual contact.

16. The ostomy device component according to any of claims 11 to 15, wherein the second portion comprises a tubular portion.

17. The ostomy device component according to claim 16, wherein the tubular portion is deformable between an axially collapsed condition and an axially extended condition.

18. The ostomy device component according to any of claims 11 to 17, wherein the second portion has a concertina bellows shape.

19. The ostomy device component according to any of claims 11 to 18, wherein the first portion comprises a ring shaped member.

20. The ostomy device component according to any of claims 11 to 19, wherein the first portion is more rigid than the second portion.

21. The ostomy device component according to any of claims 11 to 20, wherein the component comprises two of said first portions spaced apart from each other.

22. The ostomy device component according to any of claims 11 to 21, wherein the component defines a collection chamber for human waste.
